# FASCICULE DE BREVET EUROPEEN

(11) **EP 2 758 013 B1**
(45) Date de publication et mention de la délivrance du brevet: **09.03.2016**
(21) Numéro de dépôt: 12787756.1
(22) Date de dépôt: 24.09.2012
(51) Int. Cl.: A61F 9/00

(54) **DISPOSITIF DE DISTRIBUTION DE LIQUIDE SOUS FORME DE GOUTTES**
VORRICHTUNG ZUR AUSGABE EINER FLÜSSIGKEIT IN TROPFENFORM
DEVICE FOR DISTRIBUTING LIQUID IN THE FORM OF DROPS

(30) Priorité: 22.09.2011 FR 1158469
(43) Date de publication de la demande: 30.07.2014
(73) Titulaire: NEMERA LA VERPILLIERE, 38290 La Verpillière (FR)
(72) Inventeur: JULIA, Xavier, F-38090 Villefontaine (FR); PAINCHAUD, Gaetan, F-69340 Francheville (FR); GREVIN, Guillaume, F-38080 L'isle D'abeau (FR)
(74) Mandataire: Thiollier, Clémence-Olivia Laure Marie
(86) Numéro de dépôt international: PCT/FR2012/052127
(87) Numéro de publication internationale: WO 2013/041819

(56) Documents cités:
- WO-A1-2004/013009
- WO-A1-2008/068911
- DE-B3-102009 048 476
- US-A- 5 373 964
- US-A1- 2002 016 576
- US-A1- 2008 039 807

## Description

La présente invention concerne la distribution de liquide, plus particulièrement la distribution sous forme de gouttes dans le domaine pharmaceutique, par exemple du liquide ophtalmique, nasal, buccal ou auriculaire.

On entend par « produit liquide» un produit non solide et non gazeux, plus ou moins visqueux.

On connaît déjà des dispositifs de distribution comprenant un orifice de distribution d'où sortent les gouttes, ménagé par exemple au centre et à l'extrémité d'une buse de distribution, généralement de couleur blanche.

Il se trouve que la distribution de gouttes requiert une certaine précision de la part de l'utilisateur, tout particulièrement dans le cas de la distribution d'un liquide dans l'oeil.

Ainsi, une manipulation maladroite de l'utilisateur peut facilement conduire au dépôt de la goutte à côté de la zone visée.

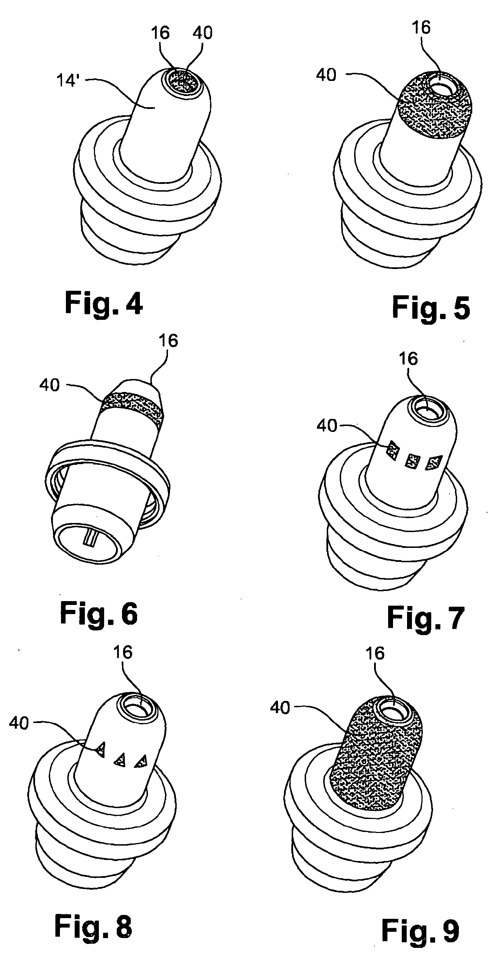

Le document US2008039807 A1 présente un dispositif (1) comprenant une loupe (3) et un embout (2) qui vient se fixer directement sur un flacon ophtalmique (figures 7A et 7B). Ledit embout possède 3 anneaux colorés: un blanc (20), une vert (22) et un rouge (23). Lesdits anneaux, en combinaison avec la loupe, permettent de savoir si l'embout est placé trop près, trop loin ou désaxé par rapport à une position et une orientation optimales (figures 6A-6C). Les zones colorées sont réalisées en tampographie.

La présente invention vise à proposer un dispositif de distribution selon les revendications déposées permettant à l'utilisateur de distribuer les gouttes avec plus de précision.

A cet effet, l'invention a pour objet un dispositif de distribution de liquide sous forme de gouttes comprenant un orifice de distribution des gouttes ménagé sur une extrémité de distribution, dans lequel le dispositif comprend des moyens de localisation visuelle de l'orifice, par réalisation d'une zone de démarcation ayant une couleur très contrastée par rapport à d'autres parties de l'extrémité de distribution.

Grâce à la zone de démarcation, l'utilisateur détecte très facilement où se trouve l'orifice de distribution, ce qui lui permet de mieux viser lors de la délivrance de la goutte. Cette zone de démarcation se révèle particulièrement intéressante pour la distribution de liquide dans l'oeil (liquide ophtalmique) du fait qu'au moment de la délivrance de la goutte, le dispositif se trouve extrêmement proche de l'oeil qui de ce fait, ne peut pas focaliser ou accommoder et voit le dispositif de façon très floue, sans distinguer nettement le relief du dispositif, donc l'endroit d'où la goutte va sortir. En outre dans le cas de la distribution de plusieurs gouttes consécutives dans l'oeil, l'utilisateur voit encore plus flou après la première goutte reçue.

Ainsi, au lieu de réaliser un dispositif dans lequel toutes les parties ont une couleur similaire, généralement blanche, sans contraste entre elles, les inventeurs ont eu l'idée de réaliser un fort contraste visuel pour aider l'utilisateur à localiser l'orifice, même lorsqu'il voit flou. Des tests auprès d'utilisateurs ont permis de vérifier que la précision de la distribution est nettement améliorée.

On notera que l'on entend par « orifice de distribution » la partie de l'extrémité d'où sortent les gouttes, de préférence une partie de forme conique permettant de former les gouttes, disposée au centre et en partie supérieure de l'extrémité de distribution. Par ailleurs on comprend que la zone de démarcation ayant une couleur très contrastée par rapport à d'autres parties de l'extrémité de distribution peut signifier par exemple que la zone de démarcation est très foncée alors que le reste de l'extrémité de distribution est, au moins en partie, très clair ou bien que la zone de démarcation est très claire alors que le reste de l'extrémité de distribution est, au moins en partie, très foncé.

L'invention peut en outre comporter l'une ou plusieurs des caractéristiques suivantes, prises seules ou en combinaison.
- La zone de démarcation est réalisée à l'intérieur de l'orifice de distribution. En particulier, la zone de démarcation est une zone foncée disposée dans ou à l'intérieur d'une cavité de formation des gouttes, alors que l'entourage immédiat extérieur de cette cavité de formation des gouttes est réalisé dans une couleur claire.
- La zone de démarcation est disposée autour de l'orifice de distribution. En particulier, la zone de démarcation est une zone claire disposée dans ou à l'intérieur de l'orifice de distribution, délimité de préférence par une cavité conique de formation de gouttes, alors que l'entourage immédiat de l'orifice de distribution est réalisé dans une couleur foncée. Par exemple, la zone de démarcation est réalisée sous forme d'un anneau coloré, continu ou discontinu, disposé autour de l'orifice de distribution. L'anneau coloré peut être discontinu, sous forme d'une ligne circulaire hachée, par exemple par un alignement circulaire de carrés, de triangles, de ronds ou de tout autre motif.
- Le dispositif comprend des moyens de formation de gouttes et la zone de démarcation est obtenue par coloration de ces moyens de formation de gouttes, de préférence dans une couleur foncée. Ces moyens de formation de gouttes sont de préférence composés par une forme évasée conique. Selon un mode de réalisation intéressant, les moyens de formation de gouttes sont réalisés dans une partie formant valve de passage du liquide, en matériau élastomère. De façon alternative, la zone de démarcation est disposée autour des moyens de formation de gouttes, par exemple par une coloration foncée de tout ou partie d'un capot entourant les moyens de formation de goutte.
- Le dispositif comprend un embout de distribution de forme générale sensiblement cylindrique, l'extrémité de distribution ayant une forme générale de disque au centre duquel se trouve l'orifice de distribution. Ce mode de réalisation est particulièrement intéressant du fait que, lorsque l'extrémité du dispositif se présente sous forme d'une surface sensiblement plane et relativement étendue par rapport à la surface de l'orifice de distribution, à la différence d'une buse conique protubérante, il est plus difficile pour l'utilisateur de distinguer l'orifice de distribution formé au centre de cette surface, tout particulièrement si les gouttes sont distribuées au voisinage de l'oeil. En outre, un tel embout sensiblement cylindrique peut avoir une dimension relativement réduite dans la direction longitudinale du dispositif, si bien que le dispositif est plus près de l'oeil de l'utilisateur, d'où une plus grande difficulté pour focaliser et pour voir l'embout en trois dimensions (du fait que l'embout présente moins d'ombres), donc pour voir l'embout de façon nette. L'utilisation des moyens de localisation peut donc créer un sentiment de sécurité chez l'utilisateur.
- Le disque est de couleur opaque. En d'autres termes, il est de couleur non transparente, par exemple de couleur blanche ou d'une autre couleur, ou encore présentant des motifs. Dans ce cas-là, le dispositif proposé ci-dessus est particulièrement intéressant car, lorsque lé haut du dispositif est opaque et qu'il présente un disque étendu à son extrémité proche de l'oeil, il constitue une gêne supplémentaire pour l'utilisateur, car la focalisation est plus difficile que dans le cas où le disque serait transparent.
- L'embout comprend une valve de distribution de gouttes, un support de valve rapporté sur un réservoir et un capot de plaquage de la valve contre le support, le capot portant de préférence la zone de démarcation. Par exemple, le capot a une forme générale de disque percé en son centre, muni d'une jupe annulaire venant pincer la valve contre le support. Toutefois, le dispositif de distribution peut prendre bien d'autres formes. En particulier, le dispositif peut.être un dispositif sans valve, pour la distribution de liquide sous forme de gouttes, notamment pour la distribution de collyre. Il peut également prendre la forme d'une pompe ou encore d'une valve.
- La zone de démarcation est réalisée par sérigraphie, tampographie (ou impression au tampon), marquage à chaud, impression par jet d'encre, ou encore par marquage laser.
- Le dispositif est configuré pour la distribution de liquide dans un oeil.

L'invention a par ailleurs pour objet l'utilisation d'un dispositif tel que décrit ci-dessus pour la distribution de liquide dans un oeil.

L'invention a également pour objet un procédé de fabrication selon les revendications déposées d'un dispositif tel que décrit ci-dessus, comprenant une étape de marquage afin de réaliser la zone de démarcation, par exemple une étape de sérigraphie, de tampographie, de marquage à chaud, d'impression par jet d'encre, ou encore de marquage laser.

L'invention sera mieux comprise à la lecture de la description qui va suivre, donnée uniquement à titre d'exemple et faite en se référant aux dessins dans lesquels :
- la figure 1 est une vue en perspective d'un dispositif selon un mode de réalisation,
- la figure 2 est une vue similaire à la figure 2, selon une variante de réalisation,
- la figure 3 est une vue en coupe longitudinale du dispositif de la figure 1, muni d'un capuchon de protection,
- la figure 4 est une vue perspective d'un dispositif selon un deuxième mode de réalisation,
- les figures 5 à 9 sont des vues en perspectives de variantes du dispositif de la figure 4.

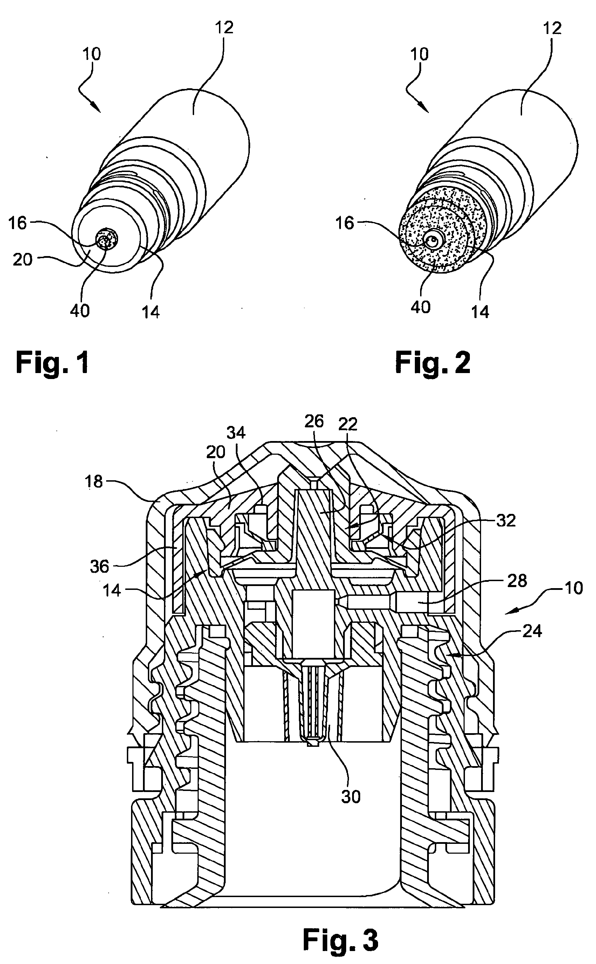

On a représenté sur la figure 1 un dispositif 10 de distribution de liquide sous forme de gouttes comprenant un réservoir 12 sur lequel est rapporté un embout de distribution 14, qui peut être surmonté d'un capuchon de protection 18, visible sur la figure 3.

L'embout 14 présente une extrémité supérieure de distribution 20 sur laquelle est ménagé un orifice 16 de distribution des gouttes délimité, dans cet exemple, par des moyens 16 de formation de gouttes. Plus précisément, toujours dans cet exemple, l'embout 14 est de forme générale sensiblement cylindrique, l'extrémité de distribution 20 ayant une forme générale de disque au centre duquel se trouve l'orifice de distribution 16. Cette extrémité 20 de forme de disque est de couleur non transparente, elle est par exemple blanche ou d'une autre couleur unie, si bien qu'elle représente une gêne pour l'utilisateur qui la rapproche très près de son oeil. Comme on peut le voir sur la figure 3, l'embout 14 comprend une valve 22 de distribution de gouttes, réalisée en matériau élastomère de façon à prendre, sous la pression du liquide (lorsque l'utilisateur appuie sur le réservoir 12), une configuration de passage du liquide. Les moyens 16 de formation de gouttes sont ménagés à l'extrémité de la valve 22, directement dans la matière élastomère. Ces moyens 16 sont de forme évasée conique. L'embout 14 comprend par ailleurs un support de valve 24, rapporté sur le réservoir 12, portant un pion 26 formant un siège contre lequel la valve 22 est plaquée et comportant également un conduit 28 de passage d'air, obturé par un organe 30 perméable à l'air empêchant l'introduction de bactéries dans le réservoir. L'embout 14 comporte également un élément de rappel 32, permettant de rappeler la valve 22 en configuration de blocage du liquide. Enfin, l'embout 14 comprend un capot 34 de plaquage de la valve contre le support 24, le capot comprenant l'extrémité 20, sous forme d'un disque sensiblement plat percé en son centre, ce disque étant muni d'une jupe annulaire extérieure 36, fixée sur le support 24, et d'une jupe intérieure de guidage de la valve 22.

Comme on peut le voir sur la figure 1, le dispositif comprend des moyens 40 de localisation visuelle de l'orifice, confondus ici avec les moyens 16 de formation de gouttes, par réalisation d'une zone de démarcation 40 ayant une couleur très contrastée par rapport à d'autres parties de l'extrémité de distribution. Dans cet exemple, la zone de démarcation 40 est réalisée à l'intérieur de l'orifice de distribution 16, par coloration des moyens de formation de gouttes. La coloration est très contrastée par rapport au reste de l'embout. Selon un exemple non limitatif, les moyens de formation de gouttes sont de couleur apparente foncée, éventuellement bleue, alors que le reste de l'embout est de couleur claire, blanche.

On notera que l'orifice de distribution 16 est réalisé sensiblement dans le même plan que la surface 20, si bien qu'il peut être difficile de le distinguer par rapport au reste de la surface 20 lorsque le dispositif 10 est disposé près de l'oeil. Aussi, il est particulièrement utile de prévoir une zone de démarcation 40. On relève en particulier que la surface 20 est relativement étendue par rapport à la surface de l'orifice 16 de distribution de gouttes : la surface 20 a une aire supérieure à l'aire de l'orifice 16, en l'occurrence plus de deux, voire trois, fois supérieure.

Dans l'exemple de la figure 2, la zone de démarcation comprend la surface supérieure 20 de l'embout, du fait que le capot 34 est de couleur foncée, bleue, et que les moyens 16 de formation de gouttes sont de couleur claire, blanche ou transparente.

Dans le mode de réalisation de la figure 4, l'embout 14' est différent de l'embout 14 en ce qu'il se présente sous forme d'une buse de distribution formant nettement saillie du reste de l'embout. Aussi, dans ce mode de réalisation, la surface supérieure du dispositif est le sommet de la buse et est quasiment constituée par l'orifice de distribution de gouttes. La zone de démarcation 40 est réalisée ici par coloration, en foncé, de moyens de formation de gouttes 16. Dans cet exemple, toute la partie de formation de gouttes est colorée, toutefois on pourrait prévoir de la colorer partiellement.

Plusieurs variantes de zones de démarcation 40 sur le dispositif de la figure 4 peuvent être envisagées, de façon non limitative. Sur la figure 5, la zone de démarcation 40 est disposée autour de l'orifice de distribution 16, sous forme d'un hémisphère de couleur foncée, l'orifice de distribution 16 étant quant à lui réalisé dans une couleur claire. Sur la figure 6, la zone de démarcation 40 est également disposée autour de l'orifice de distribution 16, sous forme d'un anneau coloré continu de couleur foncée, l'orifice de distribution 16 étant réalisé dans une couleur claire. Cet anneau coloré peut être ménagé sur une partie intermédiaire de la buse dans un mode de réalisation qui n'est pas revendiqué, ou bien au voisinage immédiat de l'orifice de distribution, de sorte que l'anneau coloré soit disposé sur la buse juste à la périphérie de moyens de formation de gouttes. Sur la figure 7, la zone de démarcation 40 est similaire à celle de la figure 6, hormis le fait que l'anneau coloré est discontinu, par alignement circulaire de carrés ou de rectangles, toujours de couleur foncée. Sur la figure 8, de même que sur la figure 7, l'anneau 40 est discontinu, formé par un alignement circulaire de triangles. Enfin, sur la figure 9, la zone de démarcation 40 est composée par toute la buse de distribution, qui est réalisée de couleur foncée, alors que seul l'orifice de distribution 16 est réalisé de couleur claire.

On notera que, selon les modes de réalisation, la zone de démarcation de couleur foncée peut être réalisée par un matériau teinté dans la masse. De façon alternative, elle est réalisée par sérigraphie, marquage à chaud, impression par jet d'encre, ou encore par marquage laser, de façon à apposer une couleur de préférence foncée sur l'extrémité de distribution. Ainsi selon un mode de réalisation, le procédé de fabrication du dispositif 10 comprend une étape de marquage afin de réaliser la zone de démarcation, par exemple une étape de sérigraphie, de tampographie, de marquage à chaud, d'impression par jet d'encre, ou encore de marquage laser.

Le dispositif 10 décrit ci-dessus est tout particulièrement avantageux lorsqu'il est configuré ou utilisé pour la distribution de liquide dans un oeil.

L'invention n'est pas limitée aux exemples décrits ci-dessus. On comprend en particulier que toute façon de réaliser une démarcation de l'orifice 16 ou de son voisinage, par contraste visuel, est envisageable, de façon à aider l'utilisateur à localiser l'orifice, tout particulièrement lorsque l'utilisateur voit flou.

On comprend par ailleurs que les caractéristiques décrites peuvent être appliquées à d'autres dispositifs de distribution que ceux décrits en exemple, tant que le dispositif est couvert par les revendications déposées.

## Revendications

1. Dispositif (10) de distribution de liquide sous forme de gouttes comprenant un orifice (16) de distribution des gouttes ménagé sur une extrémité de distribution, **caractérisé en ce que** le dispositif comprend des moyens (40) de localisation visuelle de l'orifice, par réalisation d'une zone de démarcation (40) ayant une couleur très contrastée par rapport à d'autres parties de l'extrémité de distribution, le dispositif comprenant des moyens (16) de formation de gouttes réalisés dans une partie formant valve de passage du liquide, en matériau élastomère, et la zone de démarcation (40) étant obtenue par coloration de ces moyens de formation de gouttes.

2. Dispositif selon la revendication précédente, dans lequel la zone de démarcation (40) est réalisée à l'intérieur de l'orifice de distribution (16).

3. Dispositif selon la revendication 1, dans lequel les moyens de formation de gouttes sont composés par une forme évasée conique.

4. Dispositif selon l'une quelconque des revendications précédentes, comprenant un embout (14) de distribution de forme générale sensiblement cylindrique, l'extrémité de distribution (20) ayant une forme générale de disque au centre duquel se trouve l'orifice de distribution (16).

5. Dispositif selon la revendication précédente, dans lequel le disque est de couleur opaque.

6. Dispositif selon la revendication précédente, dans lequel l'embout comprend une valve (22) de distribution de gouttes, un support de valve (24) rapporté sur un réservoir (12) et un capot (34) de plaquage de la valve contre le support.

7. Dispositif selon l'une quelconque des revendications précédentes, dans lequel la zone de démarcation (40) est réalisée par sérigraphie, tampographie, marquage à chaud, impression par jet d'encre, ou encore par marquage laser.

8. Dispositif selon l'une quelconque des revendications précédentes, le dispositif étant configuré pour la distribution de liquide dans un oeil.

9. Procédé de fabrication d'un dispositif selon l'une quelconque des revendications précédentes, comprenant une étape de marquage afin de réaliser la zone de démarcation, par exemple une étape de sérigraphie, de tampographie, de marquage à chaud, d'impression par jet d'encre, ou encore de marquage laser.

## Patentansprüche

1. Vorrichtung (10) zur Ausgabe von Flüssigkeit in Tropfenform, umfassend eine Öffnung (16) zur Ausgabe der Tropfen, die an einem Ausgabeende vorgesehen ist, **dadurch gekennzeichnet, dass** die Vorrichtung Einrichtungen (40) zur visuellen Lokalisierung der Öffnung umfasst, durch Ausbildung einer Abgrenzungszone (40), die eine Farbe hat, die gegenüber anderen Teilen des Ausgabeendes sehr kontrastiert ist, wobei die Vorrichtung Mittel (16) zur Bildung von Tropfen umfasst, die in einem Teil aus Elastomermaterial ausgebildet sind, das ein Durchlassventil für die Flüssigkeit bildet, und wobei die Abgrenzungszone (40) durch Färbung dieser Mittel zur Bildung von Tropfen erhalten wird.

2. Vorrichtung nach dem vorhergehenden Anspruch, wobei die Abgrenzungszone (40) im Inneren der Ausgabeöffnung (16) ausgebildet ist.

3. Vorrichtung nach Anspruch 1, wobei die Tropfenbildungsmittel durch eine konische aufgeweitete Form gebildet sind.

4. Verfahren nach einem der vorhergehenden Ansprüche, umfassend einen Ausgabestutzen (14) allgemeiner, im Wesentlichen zylindrischer Form, wobei das Ausgabeende (20) eine allgemeine Scheibenform hat, in deren Mitte sich die Ausgabeöffnung (16) befindet.

5. Vorrichtung nach dem vorhergehenden Anspruch, wobei die Scheibe eine opake Farbe hat.

6. Vorrichtung nach dem vorhergehenden Anspruch, wobei der Stutzen ein Ventil (22) zur Ausgabe von Tropfen, eine Ventilhalterung (24), die an einem Behälter (12) angebracht ist, und eine Kappe (34) zum Anpressen des Ventils gegen die Halterung umfasst.

7. Vorrichtung nach einem der vorhergehenden Ansprüche, wobei die Abgrenzungszone (40) durch Siebdruck, Tampondruck, Heißprägung, Tintenstrahldruck oder auch durch Laseimarkierung realisiert ist.

8. Vorrichtung nach einem der vorhergehenden Ansprüche, wobei die Vorrichtung zur Ausgabe von Flüssigkeiten als Auge ausgelegt ist.

9. Verfahren zum Herstellen einer Vorrichtung nach einem der vorhergehenden Ansprüche, umfassend einen Schritt des Markierens, um die Abgrenzungszone zu realisieren, zum Beispiel einen Schritt des Siebdrucks, des Tampondrucks, der Heißprägung, des Tintenstrahldrucks oder auch der Lasermarkierung.

## Claims

1. Device (10) for distributing liquid in the form of drops comprising a drop distribution orifice (16) formed on a distribution end, **characterized in that** the device comprises means (40) for visually locating the orifice, by the production of a demarcation zone (40) having a colour that is highly contrasted relative to the other parts of the distribution end, the device comprising drop forming means (16) produced in a part forming a valve for the passage of the liquid, made of elastomer material, and the demarcation zone (40) being obtained by the colouring of these drop forming means.

2. Device according to the preceding claim, in which the demarcation zone (40) is produced inside the distribution orifice (16).

3. Device according to Claim 1, in which the drop forming means are formed by a flared tapered shape.

4. Device according to any one of the preceding claims, comprising a distribution end piece (14) of substantially cylindrical general form, the distribution end (20) having a general form of disc at the centre of which is located the distribution orifice (16).

5. Device according to the preceding claim, in which the disc is of opaque colour.

6. Device according to the preceding claim, in which the end piece comprises a drop distribution valve (22), a valve support (24) mounted on a container (12) and a cap (34) for pressing the valve against the support.

7. Device according to any one of the preceding claims, in which the demarcation zone (40) is produced by screen printing, by pad printing, by hot marking, by ink-jet printing, or even by laser marking.

8. Device according to any one of the preceding claims, the device being configured for the distribution of liquid in an eye.

9. Method for manufacturing a device according to any one of the preceding claims, comprising a marking step in order to produce the demarcation zone, for example a step of screen printing, of pad printing, of hot marking, of ink-jet printing, or even of laser marking.
